# EUROPEAN PATENT SPECIFICATION

(11) **EP 1 897 077 B1**
(45) Date of publication and mention of the grant of the patent: **16.12.2009**
(21) Application number: 06765739.5
(22) Date of filing: 13.06.2006
(51) Int. Cl.: G08B 21/04

(54) **METHOD AND APPARATUS FOR COMMUNICATING WITH BYSTANDARS IN THE EVENT OF A CATASTROPHIC PERSONAL EMERGENCY**
VERFAHREN UND VORRICHTUNG ZUM KOMMUNIZIEREN MIT BEISTEHENDEN IM FALL EINES KATASTROPHALEN PERSÖNLICHEN NOTFALLS
METHODE ET APPAREIL POUR COMMUNIQUER AVEC DES PASSANTS DANS LE CAS D'UNE URGENCE LIEE A UNE CATASTROPHE

(30) Priority: 24.06.2005 US 693645 P; 08.12.2005 US 748917 P
(43) Date of publication of application: 12.03.2008
(73) Proprietor: Koninklijke Philips Electronics N.V., 5621 BA Eindhoven (NL)
(72) Inventor: HANSEN, Kim, J., Bothell, Washington 98041-3003 (US)
(74) Representative: Schouten, Marcus Maria
(86) International application number: PCT/IB2006/051899
(87) International publication number: WO 2006/136976

(56) References cited:
- WO-A2-03/090288
- DE-A1- 4 404 539
- JP-A- 2000 152 368
- JP-A- 2004 185 582
- US-A1- 2003 069 510
- US-B1- 6 272 379

## Description

This application claims the benefit of U.S. provisional application serial no. 60/693,645 filed 06/24/2005.

This invention relates to emergency medical alert devices and, in particular, to a system including a body worn audio communication device which can provide bystanders with verbal instructions in the event of a catastrophic personal health emergency.

Medical alert devices can save a person's life when the person experiences a catastrophic medical condition in a public area. These devices are of particular use in situations that leave a patient unconscious, such as the onset of sudden cardiac arrest. These devices can respond by communicating with a system that provides it with an indication of personal health, which can be as basic as "ok" or "not ok". This indication may be manually activated, or may include automated monitoring of one or more patient attributes coupled with automated determination of the patient's state of health.

Personal emergency response devices are manually activated systems that summon aid, which can include voice or other locally generated audio instructions that would help bystanders provide emergency assistance. One such device is described in US Pat. 6,292,687 and includes a heart dysfunction reader and sensor worn by a patient on the chest or wrist. A vital sign is monitored and the sensor determines whether a sign indicates a heart dysfunction. A signal is sent to a loop process which in turn sends a signal to a personal alarm worn by the patient. The personal alarm can broadcast a synthetic or recorded voice alerting bystanders to the medical condition.

Another device is described in US Pat. Appl. 2005/0065445 which describes an implantable sensor that, upon detection of abnormal heart activity, transmits a radio frequency signal to an external receiver carried by the patient. The external receiver has multiple communication capabilities including an enunciator which may be heard by bystanders.

Another device is an attitude-activated warning device described in US Pat. 3,634,885. The device is worn by the patient and contains a mercury switch when closes if the patient collapses to a prone position. The switch closure activates an endless tape in the device which gives instructions to bystanders by means of a loudspeaker. A similar device is described in US Pat. 6,570,503. See also US Pat. Appl. 2005/0030190.

Another device is an implantable electronic system with acute myocardial infarction detection and patient warning capabilities described in US Pat. 6,272,379. The system comprises implanted internal equipment and external equipment. The internal equipment transmits signals to the external equipment in a wireless manner.

There remains a need for a body worn audio communication device which monitors a patient vital sign and issues verbal instructions to bystanders in the event of an incapacitating medical emergency. Such a device should be compact, unobtrusive, and exhibit low power consumption.

In accordance with the principles of the present invention, an audio communication device is described which improves the probability of a patient's survival by providing directions for bystanders in verbal form from a physiological sensor and processor worn on the patient's body. The simple sounding of an alarm tone might cause a bystander to pay attention to an unconscious person, but when the alarm is accompanied by a voice prompt that for example, states "This is a medical emergency. Please call for help immediately", the bystander will know that the alarm was not a cell phone ringing, but was in fact a call for assistance. Upon hearing the voice instruction, the bystander will be able to recognize the nature of the alarm and is given the next steps to follow, thus reducing overall reaction time and improving patient outcome.
The invention is defined by the independent claim. The dependent claims define advantageous embodiments.

In the drawings:
FIGURE 1 illustrates in block diagram form a medical audio communication system constructed in accordance with the principles of the present invention.
FIGURE 1a illustrates in block diagram form the power subsystem for the medical audio communication system of FIGURE 1.
FIGURE 2 illustrates a frequency modulated waveform of the type produced by the system of FIGURE 1.
FIGURE 3a illustrates a push-pull amplifier portion of the audio drive circuitry of the system of FIGURE 1.
FIGURE 3b illustrates an H-bridge amplifier portion of the audio drive circuitry of the system of FIGURE 1.
FIGURE 4 illustrates in block diagram form a second example of a medical audio communication system constructed in accordance with the principles of the present invention.
FIGURE 5 is an exploded illustration of a medical monitor and alert device constructed in accordance with the principles of the present invention.

There are numerous ways to implement an on-body emergency instruction system in accordance with the principles of the present invention. In one example described below the system comprises a device that integrates a physiological signal sensor, processor, and loudspeaker that attaches to the skin and that is unobtrusive such that a wearer is not overly burdened by its presence. Conventional audio transducers for example, tend to be relatively large and bulky if they are to be loud enough to be heard by bystanders. Headphone drivers, for example, are small but may not produce sufficient audio volume to be easily heard at a short distance. Speaker systems consisting of a motor and a moving diaphragm tend to be large, bulky, and require considerable power. These characteristics all conflict with the objective of being unobtrusive.

The following examples of the present invention provide satisfactory fidelity while emphasizing small size and power efficiency. These examples take advantage of the ability to communicate spoken information by modulation of a pulse train. One technique is to drive a piezoelectric transducer at a very low frequency, well below its natural or designed resonant frequency. One of the following examples drives the transducer by modulating the transducer's supply voltage with the envelope of the spoken words. Doing so causes the amplitude of the transducer's output to track the verbal amplitude, giving an understandable representation of the spoken message. In addition, because of a typical "cut-off" characteristic wherein the transducer will produce no sound if the supply voltage fall below a certain threshold, some frequency content can be reproduced as well. To maximize the volume of the resulting speech, the source material should be optimized in frequency content, preferably near the transducer's resonant frequency. Likewise, speech intelligibility can be optimized by altering the pace and intonations in the source material. Piezoelectric transducers that are, for instance, 2mm thick and 6mm square can produce sound pressure levels in excess of 85dB while consuming only about 20 mA of current. These levels are easily heard by bystanders. The small size permits inclusion of this piezoelectric "loudspeaker" in small, light packages that can be integrated into a body-worn monitoring system, and the inherent design of the piezoelectric transducer produces high audio volume with minimal power demand.

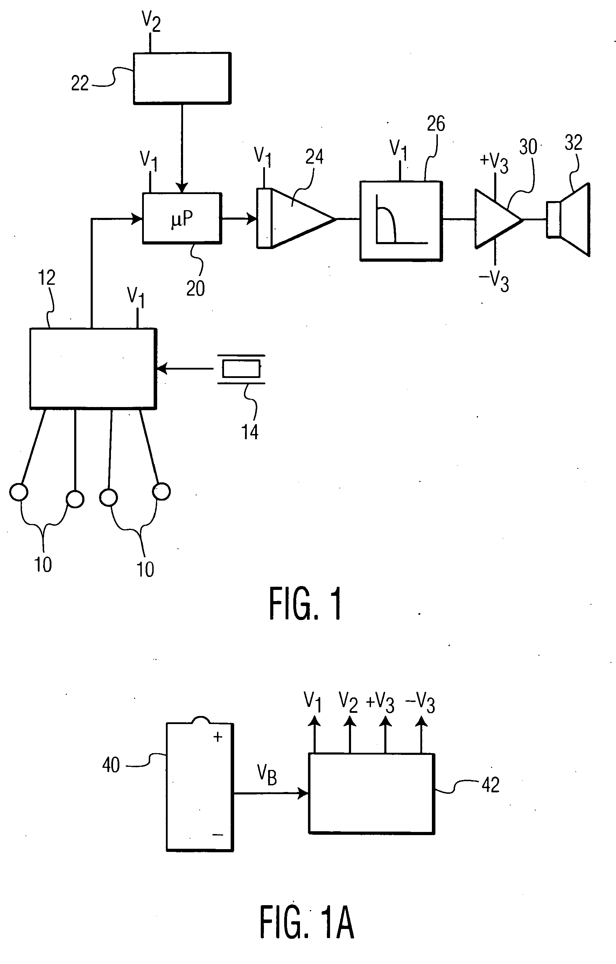

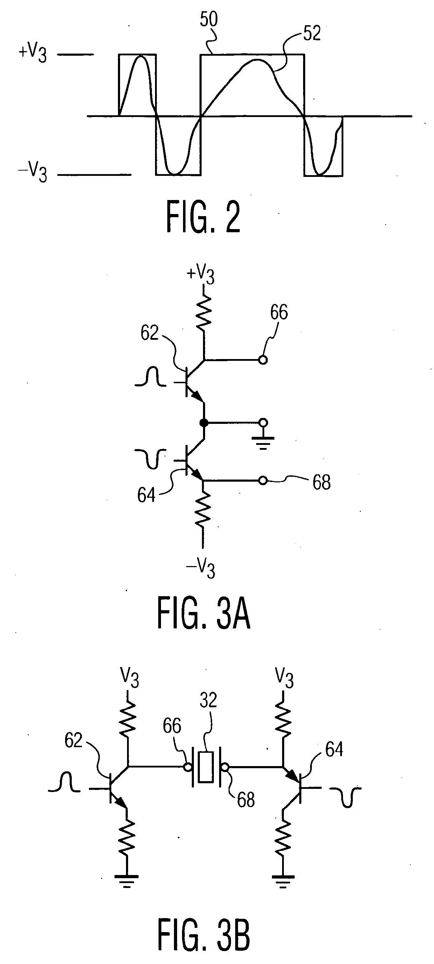

In another example, a frequency modulation technique is employed. Turning to FIGURE 1 an example of a frequency modulated system is shown. Electrodes or sensors 10 are in contact with the skin of the patient to monitor a body sign such as the heartbeat or ECG. Signals received by these sensors are sampled and digitized by a multiplexer and analog to digital converter 12. The digitally sampled signals are coupled to a microprocessor 20 where they are processed and analyzed for indication of a medical condition such as cardiac arrest. The mux and ADC 12 is also coupled to receive signals produced by a piezoelectric motion sensor 14 which can detect, for instance, whether the patient has just experienced a sudden fall as would occur if the patient suddenly became unconscious. The sensor data is also coupled to the microprocessor 20 for processing and analysis. A memory 22 is coupled to the microprocessor 20 and will generally contain program data, store patient physiological data at the time of a medical emergency for instance, and will store one or more prerecorded voice responses in accordance with the present invention. If a serious medical condition is detected by the microprocessor the microprocessor will recall the appropriate response data from the memory 22. For instance, a period of no signal from the motion sensor (the patient is still, unconscious) accompanied by arrhythmic heart signals may be interpreted by the system as the onset of cardiac arrest. An output of the microprocessor 20 is coupled to the input of a digital to analog converter 24 which produces an analog output signal from the response data which is smoothed by a subsequent low pass filter 26. The digital to analog converter 24 can have whatever resolution the designer desires and can be as simple as a one-bit D/A converter. The filtered analog signal is applied to the input of an amplifier 30 which drives a piezoelectric transducer loudspeaker 32. In a constructed implementation of this example the amplifier 30 had a complementary drive output. This can be constructed as a push-pull output amplifier as illustrated by transistors 62,64 with output terminals 66,68 in FIGURE 3a, or as an H-bridge output amplifier as illustrated in FIGURE 3b, in which the transistors 62,64 are driving piezoelectric loudspeaker 32. The transistors 62,64 are driven by complementary signals as illustrated at the base electrodes of the transistors. The output terminals 66,68 of the amplifier are coupled to the electrodes of the piezoelectric transducer 32. The output amplifier can be powered over a range of voltages such as ±3 volts to ±12 volts. Other voltages ranges may also be acceptable in a given construction.

FIGURE 2 is an example of the type of drive signal which may be produced for the piezoelectric transducer 32 in FIGURE 1. This drawing illustrates a binary pulse train 50, the form of which can be stored in the memory 22. When the pulse train 50 is converted to an analog signal, filtered, and applied over a voltage range of ±V₃, a drive signal such as 52 results. As the drawing shows, cycles of the drive signal are in proportion to the widths of segments of the binary pulse train, which in effect is a pulse width modulated signal. The pulse width modulation results in a frequency modulated signal which is used to drive the piezoelectric transducer 32.

FIGURE 1a illustrates a power subsystem which can be used to power the components of the system of FIGURE 1. A rechargeable lithium ion battery 40 provides a battery voltage V_{B} such as 3.6 volts. A lithium ion battery has a relatively low internal series resistance which, in conjunction with the use of a piezoelectric transducer for the loudspeaker, will maintain its output voltage while current is drawn from the battery. The battery voltage V_{B} supplies power for a power supply 42 which provides the necessary voltages for the system. In one constructed implementation the battery produced four supply voltages: 1.8 volts (V₁) for the low power circuitry, 3 volts (V₂) for the memory which used a flash memory device, and ±3 volts for the piezoelectric transducer drive circuitry. As mentioned above, other voltages may be used depending upon the needs of the circuitry used in the design.

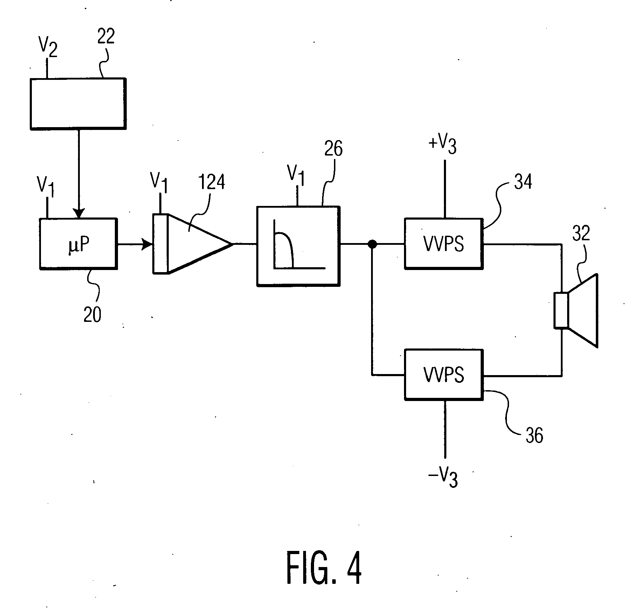

FIGURE 4 illustrates another example of the present invention in which amplitude modulation is used for the audio signal. As previously mentioned, in such an example the piezoelectric transducer is driven by modulating the transducer's supply voltage. The digitized, prerecorded voice message is accessed from the memory 22 by the microprocessor 20 and applied to an n-bit D/A converter 124. The output voltage from the D/A converter 124 is smoothed by a lowpass filter 26 and applied to the reference voltage inputs of two voltage variable power supplies 34 and 36, which are complementarily powered by supply voltages +V₃ and -V₃. The outputs of the voltage variable power supplies 34 and 36 are coupled to the electrodes of the piezoelectric transducer 32, driving the transducer with a voltage that varies in the range of ±V₃. Some users may prefer the voice clarity of this amplitude modulated technique over the frequency modulated technique described above.

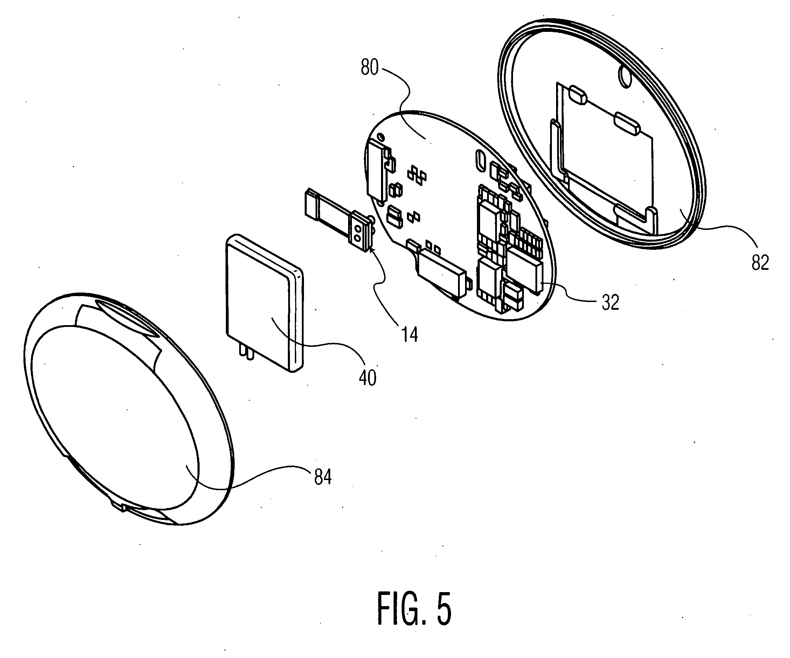

FIGURE 5 is an exploded view of a body-worn sensor which is small, light, and attaches to the body by a conductive adhesive. The sensor has a clamshell case of two halves 82 and 84. On the outside of the case half 82 are electrodes coated with a conductive adhesive that attaches the sensor to the body and conducts vital sign signals to the electrodes. The electrical components of the sensor are located on a printed circuit assembly 80, including the piezoelectric motion sensor 14. The battery 40 is located between the printed circuit assembly and the case half 84. The piezoelectric transducer 32 may be located on the printed circuit assembly 80 as shown, or may be attached to case half 84 to take advantage of the acoustic properties of the case and better transmit the voice message to bystanders.

## Claims

1. A body-worn communicator of messages regarding a medical emergency experienced by a wearer comprising:
a vital sign sensor (10, 12) coupled to the wearer;
a vital sign signal processor (20);
a source of a prerecorded message (22);
an amplifier (30) coupled to receive a prerecorded message from the source and having a drive signal output; and
a piezoelectric transducer (32) coupled to the drive signal output which produces an audio message;
wherein the piezoelectric transducer (32) is driven by modulating the transducer's supply voltage with the envelope of the prerecorded message (22).

2. The body-worn communicator of Claim 1, wherein the audio message comprises a voice message.

3. The body-worn communicator of Claim wherein the audio message comprises an alarm tone.

4. The body-worn communicator of Claim 1, wherein the vital sign sensor (10, 12) comprises an arrhythmia sensor.

5. The body-worn communicator of Claim 4, wherein the arrhythmia sensor comprises an ECG sensor.

6. The body-worn communicator of Claim 1, further comprising a single power source (40, 42) which acts to power the vital sign sensor (10, 12), the prerecorded message source (22), and the amplifier (30).

7. The body-worn communicator of Claim 6, wherein the power source comprises a battery (40) producing a voltage of 12 volts or less.

8. The body-worn communicator of Claim 7, further comprising a case (82, 84) containing the vital sign signal processor (10, 12), the prerecorded message source (22), the amplifier (30), the battery (40), and the piezoelectric transducer (32),
wherein at least a portion of the vital sign sensor (10, 12) is located on the outside of the case (82, 84).

9. The body-worn communicator of Claim 7, wherein the battery (40) comprises a lithium ion battery.

10. The body-worn communicator of Claim 4, wherein the vital sign sensor (10, 12) further comprises a motion sensor (14) coupled to the vital sign signal processor.

11. The body-worn communicator of Claim 1, wherein the amplifier (30) further comprises a voltage variable power supply.

12. The body-worn communicator of Claim 11, wherein the amplifier (30) further comprises a pair of voltage variable power supplies (34, 36) coupled to the piezoelectric transducer (32).

13. The body-worn communicator of Claim 1, wherein the amplifier (30) exhibits a complementary drive output.

14. The body-worn communicator of Claim 13, wherein the complementary drive output comprises a push-pull output amplifier (62, 64).

15. The body-worn communicator of Claim 13, wherein the complementary drive output comprises an H-bridge output amplifier (62, 64).

16. The body-worn communicator of Claim 1, wherein the source of a prerecorded message (22) comprises a digital memory device.

17. The body-worn communicator of Claim 8, further comprising means for attaching the case (82, 84) to the body of a patient with the vital sign sensor (10, 12) in communication with the skin surface of the patient.

18. The body-worn communicator of Claim 17, wherein the means for attaching further comprises an adhesive gel.

## Patentansprüche

1. Am Körper getragener Kommunikator für Mitteilungen bezüglich eines durch einen Träger erlittenen medizinischen Notfalls, wobei der Kommunikator Folgendes umfasst:
einen mit dem Träger verbundenen Vitalparametersensor (10, 12);
einen Vitalparametersignalprozessor (20);
eine Quelle einer zuvor aufgezeichneten Mitteilung (22);
einen Verstärker (30), der verbunden ist, um eine zuvor aufgezeichnete Mitteilung von der Quelle zu empfangen und der einen Ansteuerungssignalausgang hat; und
einen piezoelektrischen Wandler (32), der mit dem Ansteuerungssignalausgang verbunden ist und eine Audiomitteilung erzeugt; wobei der piezoelektrische Wandler (32) angesteuert wird, indem die Versorgungsspannung des Wandlers mit der Hüllkurve der zuvor aufgezeichneten Mitteilung (22) moduliert wird.

2. Am Körper getragener Kommunikator nach Anspruch 1, wobei die Audiomitteilung eine Sprachmitteilung umfasst.

3. Am Körper getragener Kommunikator nach Anspruch 1, wobei die Audiomitteilung einen Alarmton umfasst.

4. Am Körper getragener Kommunikator nach Anspruch 1, wobei der Vitalparametersensor (10, 12) einen Arrhythmiesensor umfasst.

5. Am Körper getragener Kommunikator nach Anspruch 4, wobei der Arrhythmiesensor einen EKG-Sensor umfasst.

6. Am Körper getragener Kommunikator nach Anspruch 1, weiterhin mit einer einzelnen Versorgungsquelle (40, 42), die dazu dient, den Vitalparametersensor (10, 12), die Quelle für zuvor aufgezeichnete Mitteilungen (22) und den Verstärker (30) zu versorgen.

7. Am Körper getragener Kommunikator nach Anspruch 6, wobei die Versorgungsquelle eine Batterie (40) umfasst, die eine Spannung von 12 Volt oder weniger erzeugt.

8. Am Körper getragener Kommunikator nach Anspruch 7, weiterhin mit einem Gehäuse (82, 84), das den Vitalparamatersignalprozessor (10, 12), die Quelle für zuvor aufgezeichnete Mitteilungen (22), den Verstärker (30), die Batterie (40) und den piezoelektrischen Wandler (32) enthält,
wobei sich zumindest ein Teil des Vitalparametersensors (10, 12) auf der Außenseite des Gehäuses (82, 84) befindet.

9. Am Körper getragener Kommunikator nach Anspruch 7, wobei die Batterie (40) eine Lithiumionenbatterie umfasst.

10. Am Körper getragener Kommunikator nach Anspruch 4, wobei der Vitalparametersensor (10, 12) weiterhin einen mit dem Vitalparametersignalprozessor verbundenen Bewegungssensor (14) umfasst.

11. Am Körper getragener Kommunikator nach Anspruch 1, wobei der Verstärker (30) weiterhin ein Stromversorgung mit variabler Spannung umfasst.

12. Am Körper getragener Kommunikator nach Anspruch 11, wobei der Verstärker (30) weiterhin ein Paar Stromversorgungen mit variabler Spannung (34, 36) umfasst, die mit dem piezoelektrischen Wandler (32) verbunden sind.

13. Am Körper getragener Kommunikator nach Anspruch 1, wobei der Verstärker (30) einen komplementären Ansteuerungsausgang aufweist.

14. Am Körper getragener Kommunikator nach Anspruch 13, wobei der komplementäre Ansteuerungsausgang einen Gegentakt-Ausgangsverstärker (62, 64) umfasst.

15. Am Körper getragener Kommunikator nach Anspruch 13, wobei der komplementäre Ansteuerungsausgang einen H-Brücken-Ausgangsverstärker (62, 64) umfasst.

16. Am Körper getragener Kommunikator nach Anspruch 1, wobei die Quelle einer zuvor aufgezeichneten Mitteilung (22) eine digitale Speichereinrichtung umfasst.

17. Am Körper getragener Kommunikator nach Anspruch 8, weiterhin mit Mitteln zum Befestigen des Gehäuses (82, 84) am Körper eines Patienten, wobei der Vitalparametersensor (10, 12) in Kommunikation mit der Hautoberfläche des Patienten ist.

18. Am Körper getragener Kommunikator nach Anspruch 17, wobei das Mittel zum Befestigen weiterhin ein Haftgel umfasst.

## Revendications

1. Communicateur porté sur le corps de messages concernant une urgence médicale subie par un porteur comprenant :
un capteur de signes vitaux (10, 12) couplé au porteur;
un processeur de signal de signes vitaux (20);
une source d'un message préenregistré (22) ;
un amplificateur (30) couplé pour recevoir un message préenregistré de la source et ayant une sortie de signal de commande ; et
un transducteur piézo-électrique (32) couplé à la sortie de signal de commande qui produit un message audio;
dans lequel le transducteur piézo-électrique (32) est commandé en modulant la tension d'alimentation électrique du transducteur avec l'enveloppe du message préenregistré (22).

2. Communicateur porté sur le corps selon la revendication 1, dans lequel le message audio comprend un message vocal.

3. Communicateur porté sur le corps selon la revendication 1, dans lequel le message audio comprend une sonnerie d'alarme.

4. Communicateur porté sur le corps selon la revendication 1, dans lequel le capteur de signes vitaux (10, 12) comprend un capteur d'arythmie.

5. Communicateur porté sur le corps selon la revendication 4, dans lequel le capteur d'arythmie comprend un capteur d'électrocardiogramme.

6. Communicateur porté sur le corps selon la revendication 1, comprenant en outre une source d'alimentation électrique unique (40, 42) qui agit pour fournir l'alimentation électrique au capteur de signes vitaux (10, 12), à la source de message préenregistré (22) et à l'amplificateur (30).

7. Communicateur porté sur le corps selon la revendication 6, dans lequel la source d'alimentation électrique comprend une pile (40) produisant une tension de 12 volts ou moins.

8. Communicateur porté sur le corps selon la revendication 7, comprenant en outre un boîtier (82, 84) contenant le processeur de signal de signes vitaux (10, 12), la source de message préenregistré (22), l'amplificateur (30), la pile (40), et le transducteur piézo-électrique (32),
dans lequel au moins une partie du capteur de signes vitaux (10, 12) est situé à l'extérieur du boîtier (82, 84).

9. Communicateur porté sur le corps selon la revendication 7, dans lequel la pile (40) comprend une pile d'ions de lithium.

10. Communicateur porté sur le corps selon la revendication 4, dans lequel le capteur de signes vitaux (10, 12) comprend en outre un détecteur de mouvement (14) couplé au processeur de signal de signes vitaux.

11. Communicateur porté sur le corps selon la revendication 1, dans lequel l'amplificateur (30) comprend en outre une alimentation électrique de tension variable.

12. Communicateur porté sur le corps selon la revendication 11, dans lequel l'amplificateur (30) comprend en outre une paire d'alimentations électriques de tension variable (34, 36) couplées au transducteur piézo-électrique (32).

13. Communicateur porté sur le corps selon la revendication 1, dans lequel l'amplificateur (30) comporte une sortie de commande complémentaire.

14. Communicateur porté sur le corps selon la revendication 13, dans lequel la sortie de commande complémentaire comprend un amplificateur de sortie symétrique (62, 64).

15. Communicateur porté sur le corps selon la revendication 13, dans lequel la sortie de commande complémentaire comprend un amplificateur de sortie avec pont en H (62, 64).

16. Communicateur porté sur le corps selon la revendication 1, dans lequel la source d'un message préenregistré (22) comprend un dispositif de mémoire numérique.

17. Communicateur porté sur le corps selon la revendication 8, comprenant en outre un moyen pour attacher le boîtier (82, 84) au corps d'un patient avec le capteur de signes vitaux (10, 12) en communication avec la surface de la peau du patient.

18. Communicateur porté sur le corps selon la revendication 17, dans lequel le moyen d'attachement comprend en outre un gel adhésif.
